# EUROPEAN PATENT SPECIFICATION

(11) **EP 3 019 093 B1**
(45) Date of publication and mention of the grant of the patent: **29.08.2018**
(21) Application number: 14742109.3
(22) Date of filing: 30.06.2014
(51) Int. Cl.: A61B 17/12, A61B 17/064, A61B 17/08, A61B 17/128, A61B 17/122

(54) **TISSUE GRASPING AND WOUND CLOSING CLIPPING DEVICE**
KLEMMVORRICHTUNG ZUM GREIFEN VON GEWEBEN UND ZUM SCHLIESSEN EINER WUNDE
DISPOSITIF DE CLIPSAGE A SAISIR LES TISSUS ET POUR REFERMER UNE PLAIE

(30) Priority: 10.07.2013 US 201361844608 P
(43) Date of publication of application: 18.05.2016
(73) Proprietor: Boston Scientific Scimed, Inc., Maple Grove, MN 55311 (US)
(72) Inventor: KAPPEL, Gary, Acton, MA 01720 (US); CROWLEY, Peter, Norfolk, MA 02056 (US)
(74) Representative: Vossius & Partner Patentanwälte Rechtsanwälte mbB
(86) International application number: PCT/US2014/044933
(87) International publication number: WO 2015/006083

(56) References cited:
- WO-A1-2008/137326
- WO-A1-2009/155286
- WO-A1-2012/160562
- WO-A2-2009/136397
- US-A1- 2003 153 946
- US-A1- 2006 155 308
- US-A1- 2006 155 310
- US-A1- 2011 046 651

## Description

### Background

Pathologies of the gastrointestinal system, the biliary tree, the vascular system and other body lumens and hollow organs are often treated through endoscopic procedures, many of which require active and/or prophylactic hemostasis to control bleeding. Tools for deploying hemostatic clips via an endoscope are often used to stop internal bleeding by clamping together the edges of the wounds or incisions. Hemostasis clips grasp tissue surrounding a wound and hold edges of the wound together by applying pressure to the site to allow natural healing processes to close the wound. Specialized endoscopic clipping devices are used to deliver the clips to the desired locations within the body and to position and deploy the clips at the desired locations after which the clip delivery device is withdrawn, leaving the clip within the body. These clips may be left in place until they are sloughed off through natural processes or removed later through a separate procedure after the bleeding site has healed.

US 2006/0155310 A1 discloses clips with multiple independently-controlled legs. A device comprises an outer tube, a pusher tube assembly comprising an inner tube, and a clamp collar. A retainer is located proximally to the clamp collar with slots that allow passage of the legs.

WO 2009/136397 A2 discloses a compression clip assembly for compressing tissue. The assembly comprises a compression clip and a lock element. The clip includes a pair of normally spaced apart elongate members each having an outward-facing surface, and having respective inward-facing opposing surfaces for compressing tissue; a hinge formed at least partly of a superelastic material and in operative mechanical connection with the elongate members; and a lock region formed on the outer surface of each of the elongate members adjacent to the hinge. Each lock region is delimited by a first stop element proximal to the hinge and a second stop element distal from the hinge. The lock element is constructed for lockably engaging the lock regions so as to lock the clip in its closed position.

### Summary of the Invention

A device for clipping tissue as recited in the independent claim is provided. The dependent claims define embodiments.

The present invention is directed to a system for clipping tissue, grasping, compressing, applying hemostasis, closing wounds, etc. comprising a clip including first and second arms coupled to one another and a pusher member slidably received over a proximal portion of the device and a proximal end of the clip. The system further comprises an outer sleeve distal of the pusher member and slidably received over the first and second arms and being pushed distally over the clip by the pusher member from a first position in which the first
and second arms are unconstrained by the outer sleeve to move apart from one another to a tissue receiving configuration to a second position in which the outer sleeve surrounds and draws radially inward the first and second arms to a closed tissue gripping configuration. The system further comprises a clip holder having first and second fingers on a distal end thereof, the first and second fingers being held, before the pusher member has pushed the outer sleeve to the second position, by an inner surface of the pusher member against first and second angled cuts on a proximal end of the clip to couple the clip to the proximal portion of the device and, when the pusher member is moved distally to push the outer sleeve to the second position, being released to move radially outward releasing the clip from the proximal end of the device.

### Brief Description of the Drawings

Fig. 1 shows a perspective view of a clipping device according to a first exemplary embodiment of the present invention in an open tissue receiving configuration;
Fig. 2 shows a perspective view the clipping device of Fig. 1 in a closed tissue gripping configuration;
Fig. 3 shows a perspective view of a clip of the clipping device of Fig. 1 in an open tissue receiving configuration;
Fig. 4 shows a perspective view of a clip of the clipping device of Fig. 1 in a closed tissue gripping configuration;
Fig. 5 shows a partial cross-sectional side view of the clipping device of Fig. 1 in a first operative configuration;
Fig. 6 shows a partial cross-sectional side view of the clipping device of Fig. 1 in a second operative configuration;
Fig. 7 shows a first partial cross-sectional side view of the clipping device of Fig. 1 in a third operative configuration;
Fig. 8 shows a second partial cross-sectional side view of the clipping device of Fig. 1 in the third operative configuration;
Fig. 9 shows a partial cross-sectional side view of the clipping device of Fig. 1 in a fourth operative configuration;
Fig. 10 shows a first perspective view of a clipping device according to a second exemplary embodiment of the present invention in an open tissue receiving configuration;
Fig. 11 shows a second perspective view of the clipping device of Fig. 10 in an open tissue receiving configuration;
Fig. 12 shows a third perspective view of the clipping device of Fig. 10 in an open tissue receiving configuration;
Fig. 13 shows a perspective view of a clipping device according to a third exemplary embodiment of the present invention;
Fig. 14 shows a perspective view of the clipping device of Fig. 10 in a tissue gripping configuration;
Fig. 15 shows a perspective view of a clipping device according to a fourth exemplary embodiment of the present invention;
Fig. 16 shows a first perspective view of a band of the clipping device of Fig. 10 in an implanted configuration; and
Fig. 17 shows a second perspective view of a band of the clipping device of Fig. 10 in an implanted configuration.

### Detailed Description

The present may be further understood with reference to the following description and the appended drawings, wherein like elements are referred to with the same reference numerals. The present invention relates to devices for clipping tissue and, in particular, to a hemostasis clip that may be deployed in a single stage deployment process. The exemplary clip according to the invention extends from a proximal end connected to a clip holder to a distal end including a plurality of clip arms formed to capture tissue therebetween. An over-ring is slidable along a length of the clip arms and, in an operative configuration, lockingly engages detents formed on outer surfaces of one or more of the clip arms to lock the clip in a closed tissue gripping configuration. Specifically, once the clip has been positioned near a target portion of tissue, the clip is advanced out of a sheath and permitted to expand under a spring bias to an open tissue receiving configuration. An over-ring pusher is then advanced distally to move the over-ring distally drawing the arms radially inward to a tissue gripping configuration. As those skilled in the art will understand, when deployed to tissue surrounding a bleeding wound the clip arms draw the edges of the wound together effecting hemostasis of the target tissue site. However, as those skilled in the art will understand, these clips may be used for any application in which portions of tissue need to be drawn together. As the over-ring is pushed distally, the clip holder releases its grasp on the proximal end of the clip, deploying the clip from an insertion device. The insertion device is then removed from the body, leaving the clip in place over the target tissue. The exemplary clip according to the invention may be used for fastening tissue layers together, for closing an opening in one or more layers of tissue, for lung tissue compression, to compress bronchiole / alveoli tissue in emphysema patients, for the treatment of Chronic Obstructive Pulmonary Disease ("COPD"), etc. For example, the clipping device may be used to close wounds and/or incisions for hemostasis of natural or surgical bleeding, "stitching" a wound, occluding a vessel or lumen, plicating a hollow organ, attaching tissues, tissue approximation, etc. The term proximal, as used herein, refers to a direction approaching a physician or other user of the device while the term distal refers to a direction away from the physician or user (e.g., approaching a target portion of a tissue to be treated).

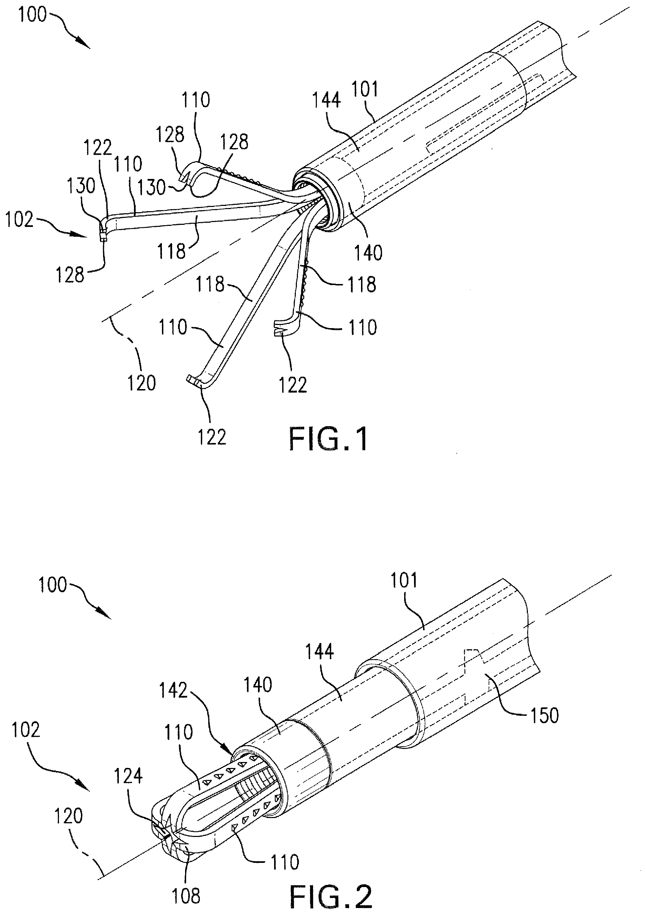

As shown in Figs. 1 - 4, a clipping device 100 according to a first exemplary embodiment of the present invention, comprises a clip 102 extending from a first end 104 having an attachment member 106 to a second end 108 having a plurality of clip arms 110. The attachment member 106 is permanently attached to the clip arms 110, which may be lockingly received within an opening 107 formed in the attachment member 106. The attachment member 106 is substantially cylindrical and includes first and second angled cuts 112, 114 formed on opposing side walls thereof and defining grooved openings on the attachment member 106. As will be described in greater detail later on, these angled cuts 112, 114 are sized, shaped and oriented to removably engage a clip holder 150. The attachment member 106 further comprises a slot 107 extending therethrough, the slot 107 permitting compression of the attachment member 106 to aid in release of the clip 102 from the clip holder, as will be described in greater detail with respect to the exemplary method below. In an exemplary embodiment, the angled cuts 112, 114 are separated from one another by 180 degrees although other angles may be used without deviating from the scope of the invention.

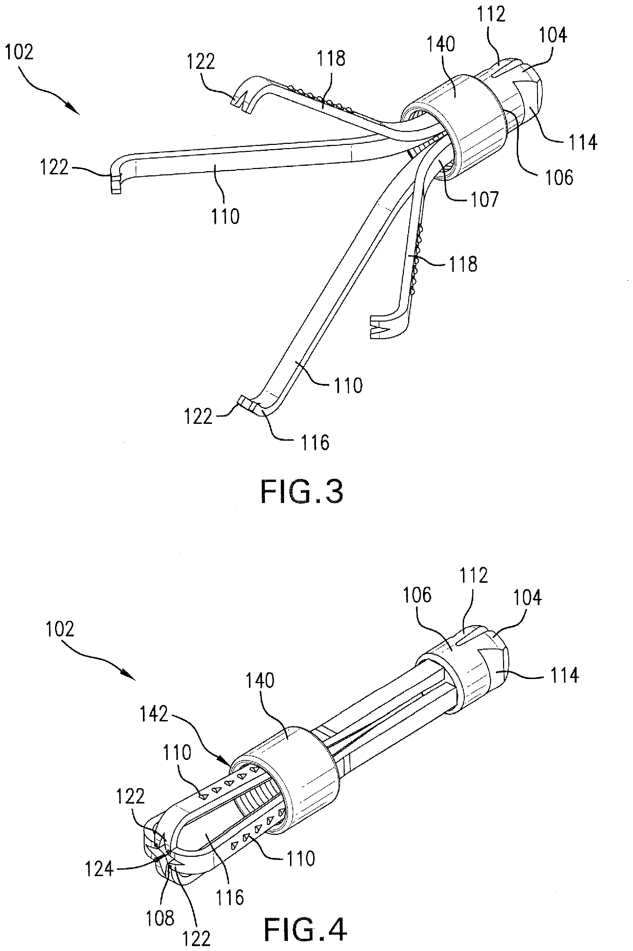

In a first exemplary embodiment, the clip 102 includes four clip arms 110, each separated from the other by 90 degrees. It is noted, however, that any number of clip arms 110 may be used without deviating from the scope of the invention and these arms may be spaced from one another by equal angles or in any other spacing as may be desired for a particular application. As those skilled in the art will understand, the use of four clip arms 110 as opposed to the conventional two clip arms aids in grasping tissue and maintaining a position of the clip 102 against the tissue for the intended period of implantation. Each of the clip arms 110 is formed with a curvature selected such that, when the clip arms 110 are in a closed position as shown in Figures 2 and 4, a tissue-receiving cavity 116 is formed between the clip arms 110. Specifically, the clip arms 110 are formed with a first angled portion 118 extending radially outward relative to a central longitudinal axis 120 of the clip 102. A second angled portion 122 of each clip arm 110 is angled radially inward such that the clip arms 110 converge toward a common end 124. Free ends 126 of the clip arms 110 are formed with a toothed shape having, for example, two barbs 128 separated from one another by a recess 130. As those skilled in the art will understand, the toothed shape helps the clip arms 110 cut into and grasp target tissue. In an exemplary embodiment, the first portions 118 of the clip arms 110 may be angled up to approximately 80 degrees relative to the central longitudinal axis 120 in the open, tissue receiving, configuration of Figure 1. As would be understood by those skilled in the art and as will be described in more detail below, the angle will depend on how far distally the clip 102 is advanced out of a sheath 101. It is noted, however, that this angle may change according to the requirements of a procedure (e.g., the size of a tissue sample to be grasped, etc.). In another embodiment, the clip arms 110 may include blunt ends or rounded ends to be less traumatic to tissue, be formed with plastic tips over their distal ends or may include any type of sharpened ends without deviating from the scope of the invention and selected to conform to the requirements of a particular procedure, as those skilled in the art will understand. The clip arms 110 are biased toward a radially expanded configuration and maintained in a closed configuration during insertion into the body due to engagement of the clip arms 110 with walls of a sheath 101 (e.g., of an endoscope).

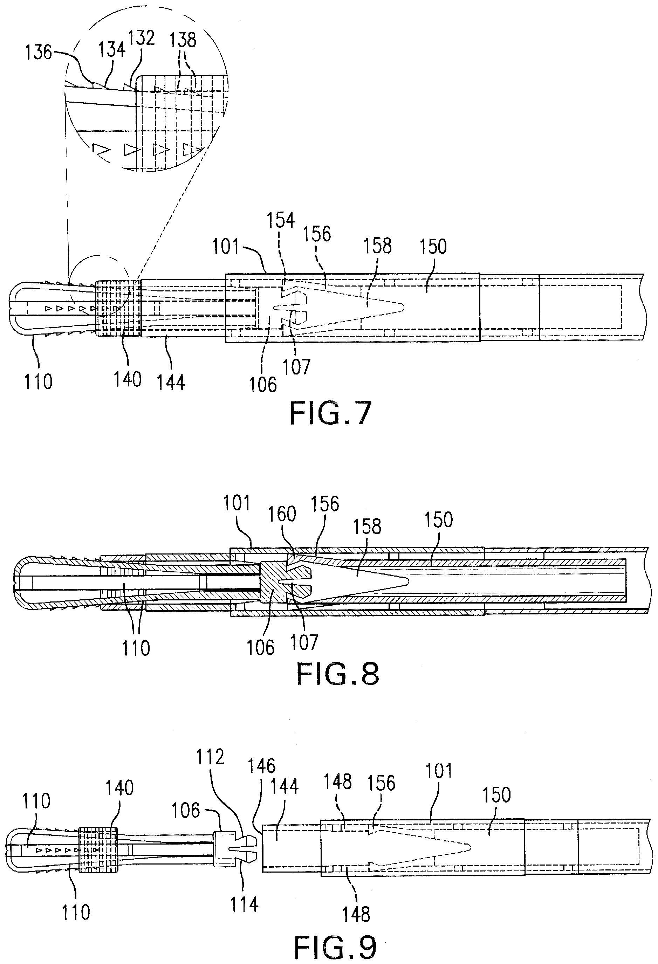

An outer surface of each of the clip arms 110 includes a plurality of detents 132 arranged parallel to the clip arm 110. As shown in greater detail in Figure 7, each of the detents 132 includes a first angled surface 134 at an angle of less than 90 degrees relative to the central longitudinal axis 120 and a second angle surface 136 extending away from the first angled surface 134 toward the clip arm 110. In a first exemplary embodiment, the second angled surface 136 is orthogonal to the central longitudinal axis 120, although any other angle may be used without deviating from the scope of the invention. For example, the second angled wall 136 may be angled within a range of, for example, 10 to 45 degrees relative to the central longitudinal axis 120. The detents 136 are formed and oriented to engage serrations 138 formed on an inside wall of a over-ring 140 which is slidably received over the clip arms 110.

The over-ring 140 is a substantially cylindrical hollow element having an opening 142 extending therethrough being sized to be slidably received over the clip arms 110 in the closed, tissue gripping configuration, as will be described in greater detail with respect to the exemplary method below. The serrations 138 are formed with angled walls corresponding to a shape of the detents 132 such that the over-ring 140 may be advanced distally over the detents 132 and then prevented from proximal retraction due to engagement of the serrations 13 8 with the detents 132, as those skilled in the art will understand. The serrations 138 may alternatively be formed as threads, knurling or another surface treated portion of the over-ring 140. In an operative configuration, the over-ring 140 is advanced distally over the clip arms 110 after capturing target tissue therebetween, the over-ring 140 locking the clip arms 110 in the tissue gripping configuration. Distal movement of the over-ring 140 is controlled via an over-ring pusher 144, as shown in Figs. 1 and 6 - 9. The over-ring pusher 144 is formed as an elongated hollow cylindrical element having a diameter substantially matching a diameter of the over-ring 140. A distal end 146 of the over-ring pusher 144 comes into contact with a proximal end 141 of the over-ring 140 to transmit distally directed force thereto. The over-ring pusher 144 may extend through an elongated shaft (e.g., endoscope) inserted into the body, a proximal end (not shown) of the over-ring pusher 144 being accessible to a user to permit actuation thereof. As those skilled in the art will understand, material properties of the over-ring pusher 144 are chosen to be substantially elastic to permit curvature thereof during insertion through tortuous paths into the body (e.g., through a body lumen accessed via a naturally occurring body orifice). The over-ring pusher 144 is sufficiently axially stiff to permit transmission of a pushing force to the over-ring 140 without buckling.

The device 100 further comprises a clip holder 150 formed as an elongated hollow cylindrical element extending from a first end (not shown) to a second end 154 having a pair of opposing fingers 156 sized and shaped to removably engage the angled cuts 112, 114 of the clip 102. The fingers 156 are defined by a pair of cutouts 158 extending through a wall of the clip holder 150. Each of the fingers 156 includes an abutting portion 160 extending radially inward by a distance selected to grip the angled cuts 112, 114. Angles of the angled cuts 112, 114 and the fingers 156 are selected to permit disengagement of the fingers 156 from the clip 102 as the clip holder 150 is retracted proximally relative to the clip 102, as will be described in greater detail with respect to the exemplary method below. In an insertion configuration, the fingers 156 are radially compressed by the over-ring pusher 144, as shown in Figure 8. The over-ring pusher 144 comprises two slots 148 formed through an outer wall thereof. The slots 148 are positioned to align with the fingers 156. When the over-ring 140 is advanced distally via the over-ring pusher 144 to move the clip 102 to the closed configuration, the slots 148 become aligned with the fingers 156, permitting radial expansion of the fingers 156 thereinto. As the clip 102 is moved distally out of the sheath 101, radial expansion of the fingers 156 along with a partial compression of the attachment member 106 due to a compressive force applied by the abutting portion 160 to the angled cuts 112, 114 releases the clip 102 from the clip holder 150. This configuration further serves as a safety mechanism, preventing release of the clip 102 from the clip holder 150 until the clip 102 has been moved to a closed configuration.

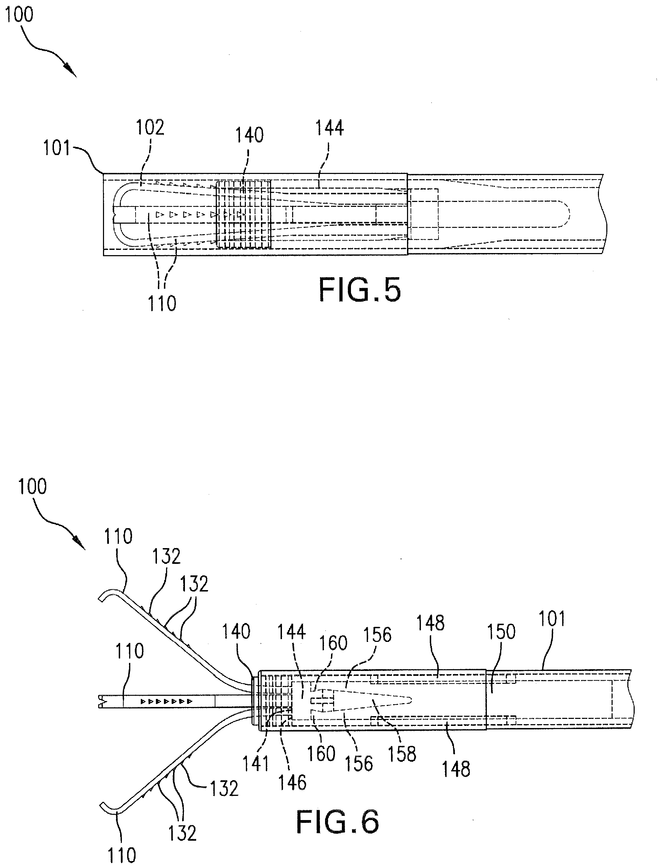

Figures 5 - 9 depict an exemplary method in which the device according to the invention may be used. In a first position, as shown in Figs. 2 and 5, the clip 102 is received within the sheath 101. In this position, the over-ring 140 may be seated over the clip arms 110 proximally of a proximal-most one of the detents 132 so that the over-ring 144 may be retracted proximally during opening of the clip 102. In a next step, as shown in Figs. 1 and 6, a first stroke is applied by the user to advance the clip holder 150 distally moving the clip 102, the over-ring 140 and the over-ring pusher 144. As the clip 102 exits the sheath 101, the clip arms 110 are radially expanded under their spring-bias. The clip 102 is then positioned over target tissue under visual or other guidance, as those skilled in the art will understand. A second stroke is then applied by the user to advance the over-ring pusher 144 distally, sliding the over-ring 140 distally over the clip arms 110 drawing the clip arms 110 together to a closed configuration over the target tissue, as shown in Figs. 7 - 8. As described in greater detail earlier, once the over-ring 140 has been pushed distally over the detents 132, the detents 132 engage the serrations 138 preventing the over-ring 140 from moving proximally. This effectively locks the clip 102 in the closed, tissue-gripping configuration. As the over-ring pusher 144 is advanced distally, the slots 148 are exposed, permitting the fingers 156 to expand radially outward, disengaging the fingers from the angled cuts 112, 114 of the clip 102 and releasing the clip 102 from the clip holder, as shown in Fig. 9. In an exemplary embodiment, the locking and releasing steps are carried out by a single stroke applied by the user. The stroke may be applied via a push button or other actuator provided on a proximal end (not shown) of the device 100. Alternatively, the device 100 may include a standard push-pull hand grip as formed in Boston Scientific Scimed devices. As those skilled in the art will understand, the device 100 may include any similar actuation means.

The device 100 may be formed to permit the firing of multiple clips 102 through a working channel of the sheath 101. Specifically, the device 100 may be removed from the working channel of the sheath 101 and the clips 102 subsequently loaded thereinto. In another embodiment, the diameters of the clips 102 may be reduced to allow multiple clips to be loaded and deployed from the working channel of the sheath 101.

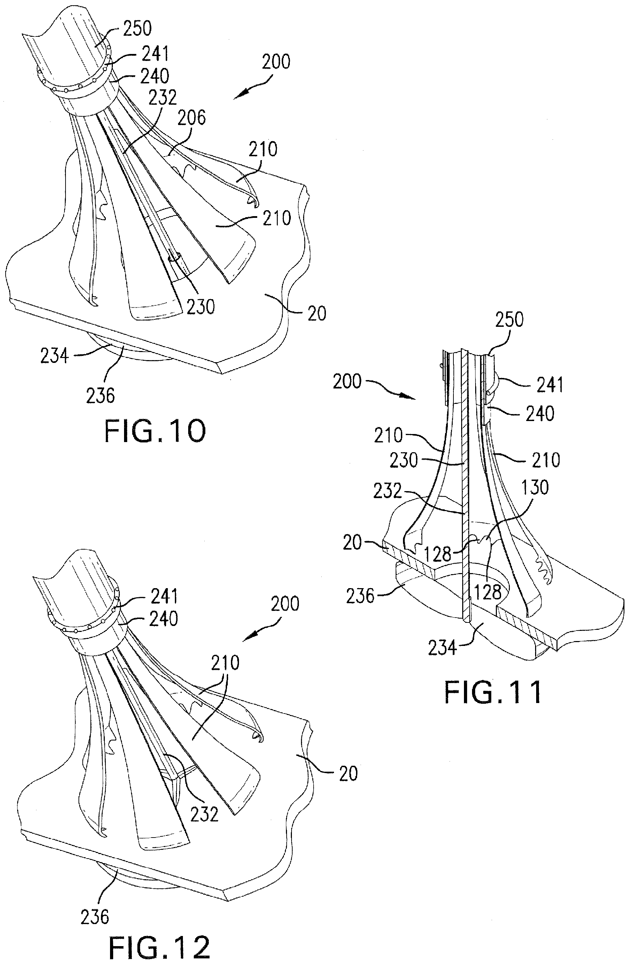

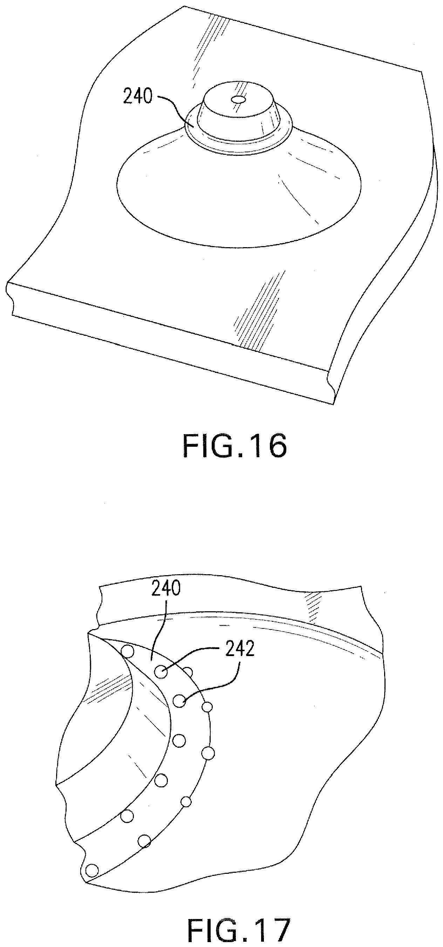

Figs. 10 - 17 depict a device 200 according to another embodiment of the invention. The device 200 is formed and operated in a manner substantially to that of the device 100, except as noted below. The device 200 includes a plurality of arms 210 spring biased toward an open tissue receiving configuration. Whereas the device 100 includes an over-ring 140 advanced distally over the clip arms 110 to lock the clip 102 in the closed configuration, the clipping device 202 includes an outer sleeve 240 advanced distally over the arms 210 to move the device 200 to the closed configuration and a band 241 stretched over the outer sleeve 240. The band 241 is pushed off a free end of the clipping device 202 after tissue has been grabbed by the arms 210 so that it contracts around this gripped tissue to, for example, cause hemostasis in the same manner as the clip arms 110 of the device 100. However, in the case of the device 200, after the band 241 has contracted around the target tissue, the arms 210 are released from the tissue and the rest of the device 200 is withdrawn from the body. Thus, as will be described in greater detail hereinafter, only the band 241 remains in the body after performing the exemplary hemostasis procedure.

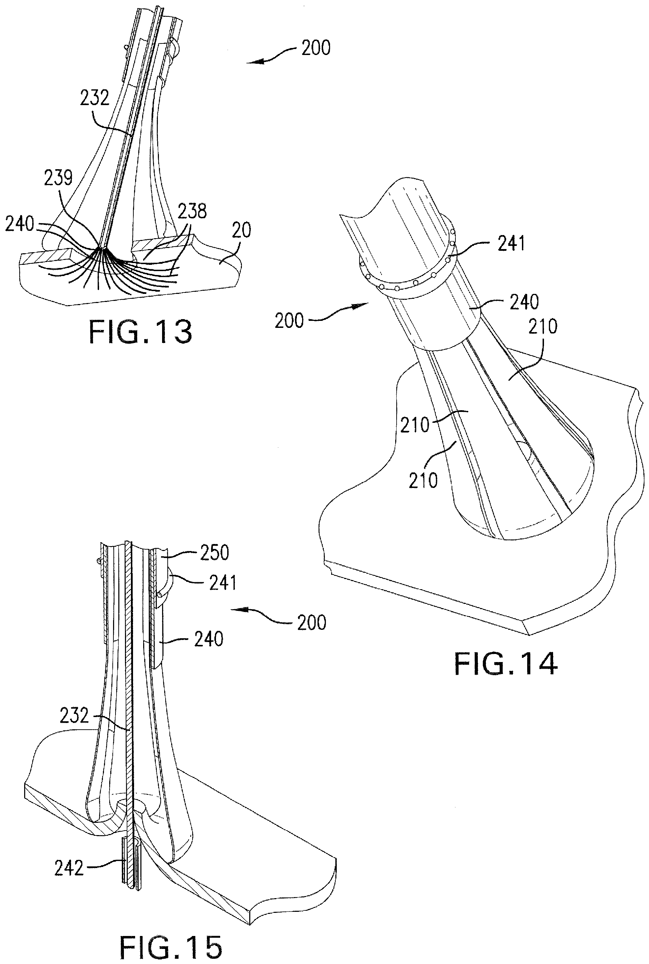

The device 200 according to the invention comprises a plurality of arms 210 biased toward an open, tissue receiving, configuration. During insertion through the body to a position adjacent to the target tissue, the arms 210 are held in a closed configuration by walls of a sheath 250 through which the clip arms 210 are inserted. As will be understood by those skilled in the art, the sheath 250 may be further received within an endoscope or other sleeve. In an exemplary embodiment, the device 200 includes six arms 210, although any other number of arms may be used without deviating from the scope of the invention. The arms 210 may be formed with any number of barbs 128 separated from one another by recesses 130 to aid in grasping tissue 20 therebetween. The number of arms 210 and respective barbs 128 as well as a shape of the barbs may be modified to conform to the requirements of a particular procedure. For example, a thickness, density and texture of the tissue 20 may warrant a stronger, sharper, duller, etc. grip, as those skilled in the art will understand. An internal support 230 is received within a cavity 206 defined by the arms 210. The internal support 230 may be used to aid in capturing tissue by providing a counter force when closing the arms 210 and may be retractable to aid in retraction of the arms 210 from the tissue. The internal support 230 may include an elongated rod 232 having an increased diameter member 234 at a distal end thereof. As shown in Figs. 10 - 12, in a first embodiment, the increased diameter member may be a plate 236 lying in a plane extending substantially perpendicular to a longitudinal axis of the device 200, where the plate 236 is formed of a material configured to expand only when positioned distally of a target tissue gripping site. In one embodiment, the plate 236 may be formed as a radially expandable balloon connected to an inflation source to permit inflation thereof once the plate 236 has been positioned in a target tissue site. As those skilled in the art will understand, the plate 236 provides a support for the arms 210 to grip and guide the tissue. In another embodiment, as shown in Figure 13, the rod 232 may be hollow and include a plurality of individual wires 238 received therein, the wires 238 expanding to an umbrella type spring support 240 as they exit a free end 239 of the rod. In yet another embodiment, as shown in Figure 15, the increased diameter member 234 may be a cylindrical member 242 formed as an increased diameter portion of the rod 232.

In accordance with an exemplary method, the device 200 is inserted into the body (e.g., along a tortuous path through a natural body lumen accessed via a body orifice) toward the target tissue 20. As shown in Figs. 10-13, the arms 210 are then advanced distally from the sheath 250 and permitted to expand under their spring bias. Once positioned over the target tissue, the outer sleeve 240 is advanced distally to draw the arms 210 closed over the tissue, as shown in Figs. 14 - 15, drawing the tissue together to seal the opening in the tissue. While the device 200 is held in place over the tissue, the band 241 is advanced distally over and beyond the outer sleeve 240 and distally off the free ends of the arms 210. The elastic band 241 then contracts over the gripped tissue maintaining compressive force thereon to maintain the hemostasis seal over the opening.

The band 241 may include any number of gripping features 242 formed to aid gripping of the tissue. In the exemplary embodiment shown, the gripping features 242 are formed as a plurality of detents or other tissue gripping features (e.g., bumps, ridges, slots that close up on
the grasped tissue, plastic spikes, etc.) on an outer surface of the band 241. It is noted, however, that any other gripping feature known in the art may be employed herein without deviating from the scope of the invention. The band 241 is formed of a polymer such as the current Boston Scientific Scimed Super 7 Speedbander® or any bioabsorbable material, as those skilled in the art will understand. In another embodiment, the tissue may be additionally sutured, clipped, cauterized or RF treated to enhance the hemostasis achieved by the band 241, as those skilled in the art will understand.

In accordance with yet another embodiment of the invention, the arms 210 may be provided on an implantable clip similar to the clip 102 and implanted in the same manner described above for the clip 102. This embodiment may employ the band 241 as an additional feature, or omitted altogether.

It will be apparent to those skilled in the art that various modifications can be made in the structure and the methodology of the present invention, without departing from the scope of the invention as defined by the claims. Thus, it is intended that the present invention cover the modifications and variations of this invention provided that they come within the scope of the appended claims and their equivalents.

## Claims

1. A device (100; 200) for clipping tissue, comprising:
a clip (102) including first and second arms (110; 210) coupled to one another;
a pusher member (144) slidably received over a proximal portion of the device (100; 200) and a proximal end of the clip (102);
an outer sleeve (140; 240) distal of the pusher member (144) and slidably received over the first and second arms (110; 210) and pushable distally over the clip (102) by the pusher member (144) from a first position in which the first and second arms (110; 210) are unconstrained by the outer sleeve (140; 240) to move apart from one another to a tissue receiving configuration to a second position in which the outer sleeve (140; 240) surrounds and draws radially inward the first and second arms (110; 210) to a closed tissue gripping configuration; **characterized in that** it further comprises a clip holder (150) having first and second fingers (156) on a distal end thereof, the first and second fingers (156) being held, before the pusher member (144) has pushed the outer sleeve (140; 240) to the second position, by an inner surface of the pusher member (144) against first and second angled cuts (112, 114) on a proximal end of the clip (102) to couple the clip (102) to the proximal portion of the device (100; 200) and, when the pusher member (144) is moved distally to push the outer sleeve (140; 240) to the second position, being released to move radially outward releasing the clip (102) from the proximal end of the device (100; 200).

2. The device of claim 1, further comprising a locking mechanism (132, 138) locking the outer sleeve (140; 240) in the second position to lock the clip (102) in the closed tissue gripping configuration, optionally further comprising first and second slots (107) in the wall of the pusher member (144) positioned to permit the first and second fingers (156) to move radially outward therein as the clip (102) is moved to the closed tissue gripping configuration to release the clip (102) from the proximal portion of the device (100; 200).

3. The device of claim 1, wherein outer walls of the first and second arms (110; 210) include a plurality of detents (132) shaped to permit sliding of the outer sleeve (140; 240) distally thereover and preventing the outer sleeve (140; 240) from moving proximally past the detents (132).

4. The device of claim 3, wherein an inner wall of the outer sleeve (140; 240) includes a plurality of serrations (138) positioned to lockingly grip the detents (132).

5. The device of claim 1,
wherein ends of the first and second arms (110; 210) include one of barbs (128), points and blunted tips.

6. The device of claim 1,
wherein the device (100; 200) further comprises third and fourth arms (110; 210), each of the plurality of arms (110; 210) being spaced circumferentially equidistant from one another and connected to one another at a common proximal end.

7. The device of claim 1, further comprising a support member (230) received within the clip (102) and including an elongated rod (232) extending to an increased diameter tip (234) at a distal end thereof, the increased diameter tip (234) extendable distally beyond distal ends of the first and second arms (110; 210) to aid in drawing tissue between the first and second arms (110; 210).

8. The device of claim 1, further comprising an elastic band (241) stretched over the outer sleeve (140; 240), the elastic band (241) being axially slidably along the clip (102) and deployed from a distal end of the clip (102) over tissue gripped by the clip (102) so that the elastic band (241) contracts around the gripped tissue.

9. The device of claim 1, wherein the first and second arms (110; 210) are biased to the tissue receiving configuration.

10. The device of any one of the preceding claims,
wherein the first and second angled cuts (112, 114) are formed on opposing side walls of an attachment member (106) of the clip (102) and define grooved openings on the attachment member (106).

11. A device for hemostasis, comprising:
a clipping device (100; 200) according to claim 1; and
a hemostasis member (241) provided on the clipping device, the hemostasis member (241) being formed to seal target tissue and maintain a seal thereon after removal of the device (100; 200) from the body.

12. The device of claim 11, wherein the hemostasis member (241) is an elastic band (241) stretched over the outer sleeve (140; 240), the elastic band (241) being axially slidably along the clipping device and deployed from a distal end of the clipping device over tissue gripped by the clipping device so that the elastic band (241) contracts around the gripped tissue.

13. The device of claim 11,
wherein the device further comprises a support member (230) received within the clip (102) and including an elongated rod (232) extending to an increased diameter tip (234) at a distal end thereof, the increased diameter tip (234) extendable distally beyond distal ends of the first and second arms (110; 210) to aid in drawing tissue between the first and second arms (110; 210), or
wherein ends of the first and second arms (110; 210) include one of barbs (128), points and blunted tips.

14. The device of claim 11, wherein outer walls of the first and second arms (110; 210) include a plurality of detents (132) shaped to permit sliding of the outer sleeve (140; 240) distally thereover and preventing the outer sleeve (140; 240) from moving proximally past the detents (132).

15. The device of claim 14,
wherein an inner wall of the outer sleeve (140; 240) includes a plurality of serrations (138) positioned to lockingly grip the detents (132).

16. The device of claim 11, wherein the first and second arms (110; 210) are spring biased to the tissue receiving configuration.

## Patentansprüche

1. Vorrichtung (100; 200) zum Klammern von Gewebe, mit:
einer Klammer (102), die erste und zweite Arme (110; 210) aufweist, die miteinander gekoppelt sind;
einem Schubelement (144), das verschiebbar über einem proximalen Abschnitt der Vorrichtung (100; 200) und einem proximalen Ende der Klammer (102) aufgenommen ist;
einer Außenhülle (140; 240) distal vom Schubelement (144), die verschiebbar über die ersten und zweiten Arme (110; 210) aufgenommen ist und durch das Schubelement (144) distal über die Klammer (102) von einer ersten Position, in der die ersten und zweiten Arme (110; 210) durch die Außenhülle (140; 240) nicht eingeschränkt sind, um sich voneinander weg in eine Gewebeaufnahmekonfiguration zu bewegen, zu einer zweiten Position schiebbar ist, in der die Außenhülle (140; 240) die ersten und zweiten Arme (110; 210) umgibt und sie radial nach innen in eine geschlossene Gewebegreifkonfiguration zieht;
**dadurch gekennzeichnet, dass** sie ferner einen Klammerhalter (150) aufweist, der erste und zweite Finger (156) an seinem distalen Ende aufweist, wobei die ersten und zweiten Finger (156), bevor das Schubelement (144) die Außenhülle (140; 240) zur zweiten Position geschoben hat, durch eine Innenfläche des Schubelements (144) gegen erste und zweite angewinkelte Einschnitte (112, 114) an einem proximalen Ende der Klammer (102) gehalten werden, um die Klammer (102) mit dem proximalen Abschnitt der Vorrichtung (100; 200) zu koppeln, und, wenn das Schubelement (144) distal bewegt wird, um die Außenhülle (140; 240) zur zweiten Position zu schieben, gelöst werden, um sich radial nach außen zu bewegen, wobei die Klammer (102) vom proximalen Ende der Vorrichtung (100; 200) gelöst wird.

2. Vorrichtung nach Anspruch 1, die ferner einen Verriegelungsmechanismus (132, 138) aufweist, der die Außenhülle (140; 240) in der zweiten Position verriegelt, um die Klammer (102) in der geschlossenen Gewebegreifkonfiguration zu verriegeln, wobei sie ferner optional erste und zweite Schlitze (107) in der Wand des Schubelements (144) aufweist, die so angeordnet sind, dass sie es den ersten und zweiten Fingern (156) ermöglichen, sich darin radial nach außen zu bewegen, wenn die Klammer (102) zur geschlossenen Gewebegreifkonfiguration bewegt wird, um die Klammer (102) vom proximalen Abschnitt der Vorrichtung (100; 200) zu lösen.

3. Vorrichtung nach Anspruch 1, wobei Außenwände der ersten und zweiten Arme (110; 210) mehrere Sperren (132) aufweisen, die so geformt sind, dass sie ein distales Verschieben der Außenhülle (140; 240) darüber ermöglichen und verhindern, dass sich die Außenhülle (140; 240) proximal an den Sperren (132) vorbei bewegt.

4. Vorrichtung nach Anspruch 3, wobei eine Innenwand der Außenhülle (140; 240) mehrere Verzahnungen (138) aufweist, die angeordnet sind, die Sperren (132) verriegelnd zu ergreifen.

5. Vorrichtung nach Anspruch 1,
wobei Enden der ersten und zweiten Arme (110; 210) Widerhaken (128) oder Spitzen oder abgestumpfte Spitzen aufweisen.

6. Vorrichtung nach Anspruch 1,
wobei die Vorrichtung (100; 200) ferner dritte und vierte Arme (110; 210) aufweist, wobei die mehreren Arme (110; 210) in Umfangsrichtung äquidistant voneinander beabstandet sind und miteinander an einem gemeinsamen proximalen Ende verbunden sind.

7. Vorrichtung nach Anspruch 1, die ferner ein Halteelement (230) aufweist, das in der Klammer (102) aufgenommen ist und einen länglichen Stab (232) aufweist, der sich zu einer Spitze (234) mit vergrößertem Durchmesser an dessen distalem Ende erstreckt, wobei die Spitze (234) mit vergrößertem Durchmesser distal über die distalen Enden der ersten und zweiten Arme (110; 210) hinaus ausfahrbar ist, um ein Ziehen von Gewebe zwischen die ersten und zweiten Arme (110; 210) zu unterstützen.

8. Vorrichtung nach Anspruch 1, die ferner ein elastisches Band (241) aufweist, das über die Außenhülle (140; 240) gespannt ist, wobei das elastische Band (241) längs der Klammer (102) axial verschiebbar ist und von einem distalen Ende der Klammer (102) über Gewebe ausgebreitet wird, das durch die Klammer (102) ergriffen wird, so dass sich das elastische Band (241) um das ergriffene Gewebe zusammenzieht.

9. Vorrichtung nach Anspruch 1, wobei die ersten und zweiten Arme (110; 210) zur Gewebeaufnahmekonfiguration vorgespannt sind.

10. Vorrichtung nach einem der vorhergehenden Ansprüche,
wobei die ersten und zweiten angewinkelten Einschnitte (112, 114) an gegenüberliegenden Seitenwänden eines Befestigungselements (106) der Klammer (102) ausgebildet sind und genutete Öffnungen am Befestigungselement (106) definieren.

11. Vorrichtung zur Blutstillung, die aufweist:
eine Klammervorrichtung (100; 200) nach Anspruch 1; und
ein an der Klammervorrichtung vorgesehenes Blutstillungselement (241), wobei das Blutstillungselement (241) ausgebildet ist, Zielgewebe abzudichten und nach der Entfernung der Vorrichtung (100; 200) von dem Körper eine Dichtung daran aufrechtzuerhalten.

12. Vorrichtung nach Anspruch 11, wobei das Blutstillungselement (241) ein elastisches Band (241) ist, das über die Außenhülle (140; 240) gespannt ist, wobei das elastische Band (241) längs der Klammervorrichtung axial verschiebbar ist und von einem distalen Ende der Klammervorrichtung über Gewebe ausgebreitet wird, das durch die Klammervorrichtung ergriffen wird, so dass sich das elastische Band (241) um das ergriffene Gewebe zusammenzieht.

13. Vorrichtung nach Anspruch 11,
wobei die Vorrichtung ferner ein Halteelement (230) aufweist, das in der Klammer (102) aufgenommen ist und einen länglichen Stab (232) aufweist, der sich zu einer Spitze (234) mit vergrößertem Durchmesser an dessen distalem Ende erstreckt, wobei die Spitze (234) mit vergrößertem Durchmesser distal über die distalen Enden der ersten und zweiten Arme (110; 210) hinaus ausfahrbar ist, um ein Ziehen von Gewebe zwischen die ersten und zweiten Arme (110; 210) zu unterstützen, oder
wobei Enden der ersten und zweiten Arme (110; 210) Widerhaken (128) oder Spitzen oder abgestumpfte Spitzen aufweisen.

14. Vorrichtung nach Anspruch 11, wobei Außenwände der ersten und zweiten Arme (110; 210) mehrere Sperren (132) aufweisen, die so geformt sind, dass sie ein distales Verschieben der Außenhülle (140; 240) darüber ermöglichen und verhindern, dass sich die Außenhülle (140; 240) proximal an den Sperren (132) vorbei bewegt.

15. Vorrichtung nach Anspruch 14,
wobei eine Innenwand der Außenhülle (140; 240) mehrere Verzahnungen (138) aufweist, die angeordnet sind, die Sperren (132) verriegelnd zu ergreifen.

16. Vorrichtung nach Anspruch 11, wobei die ersten und zweiten Arme (110; 210) zur Gewebeaufnahmekonfiguration federvorgespannt sind.

## Revendications

1. Dispositif (100 ; 200) pour la préhension de tissu, comprenant :
une pince (102) comportant un premier et un deuxième bras (110 ; 210) raccordés l'un à l'autre ;
un élément poussoir (144) monté de manière coulissante sur une partie proximale du dispositif (100 ; 200) et une extrémité proximale de la pince (102) ;
un manchon extérieur (140 ; 240) distal par rapport à l'élément poussoir (144) et monté de manière coulissante sur le premier et le deuxième bras (110 ; 210), et pouvant être poussé dans le sens distal par l'élément poussoir (144) sur la pince (102), d'une première position où le premier et le deuxième bras (110 ; 210) ne sont pas contraints par le manchon extérieur (140 ; 240) pour pouvoir s'écarter l'un de l'autre dans une configuration de réception de tissu, vers une deuxième position où le manchon extérieur (140 ; 240) entoure et ramène radialement vers l'intérieur le premier et deuxième bras (110 ; 210) dans une configuration fermée de préhension de tissu ; **caractérisé en ce qu'**il comprend en outre
un support de pince (150) ayant un premier et un deuxième doigts (156) à son extrémité distale, le premier et le deuxième doigts (156) étant maintenus, avant poussée du manchon extérieur (140 ; 240) vers la deuxième position par l'élément poussoir (144), par une surface intérieure de l'élément poussoir (144) contre une première et une deuxième découpes angulaires (112, 114) à une extrémité proximale de la pince (102) afin de raccorder la pince (102) à la partie proximale du dispositif (100 ; 200) et étant dégagés pour se déplacer radialement vers l'extérieur en débloquant la pince (102) de l'extrémité proximale de dispositif (100 ; 200) quand l'élément poussoir (144) est déplacé distalement pour pousser le manchon extérieur (140 ; 240) vers la deuxième position.

2. Dispositif selon la revendication 1, comprenant en outre un mécanisme de verrouillage (132, 138) bloquant le manchon extérieur (140 ; 240) dans la deuxième position pour maintenir la pince (102) dans la configuration fermée de préhension de tissu, comprenant en outre facultativement une première et une deuxième fentes (107) dans la paroi de l'élément poussoir (144), placées pour permettre au premier et au deuxième doigts (156) de se déplacer radialement vers l'extérieur dans celles-ci quand la pince (102) est déplacée vers la configuration fermée de préhension de tissu, afin de dégager la pince (102) de la partie proximale de dispositif (100 ; 200).

3. Dispositif selon la revendication 1, où les parois extérieures du premier et du deuxième bras (110 ; 210) présentent une pluralité de crans (132) formés pour permettre au manchon extérieur (140 ; 240) de coulisser distalement sur ceux-ci et empêcher le manchon extérieur (140 ; 240) de se déplacer proximalement au-delà des crans (132).

4. Dispositif selon la revendication 3, où une paroi intérieure du manchon extérieur (140 ; 240) présente une pluralité de dentures (138) positionnées pour retenir les crans (132) par engrènement.

5. Dispositif selon la revendication 1,
où les extrémités du premier et du deuxième bras (110 ; 210) présentent des ardillons (128) ou des pointes ou des pointes émoussées.

6. Dispositif selon la revendication 1,
où ledit dispositif (100 ; 200) comprend en outre un troisième et un quatrième bras (110 ; 210), les bras de la pluralité (110 ; 210) étant circonférentiellement espacés les uns des autres de manière équidistante et raccordés les uns aux autres à une extrémité proximale commune.

7. Dispositif selon la revendication 1, comprenant en outre un élément support (230) monté à l'intérieur de la pince (102) et comportant une tige allongée (232) s'étendant vers une extrémité évasée (234) à son extrémité distale, l'extrémité évasée (234) pouvant s'étendre distalement au-delà des extrémités distales du premier et du deuxième bras (110 ; 210) pour faciliter la traction du tissu entre le premier et le deuxième bras (110 ; 210).

8. Dispositif selon la revendication 1, comprenant en outre une bande élastique (241) étirée sur le manchon extérieur (140 ; 240), ladite bande élastique (241) pouvant coulisser axialement le long de la pince (102) et étant déployée depuis une extrémité distale de la pince (102) sur le tissu saisi par la pince (102), de sorte que ladite bande élastique (241) est resserrée autour du tissu saisi.

9. Dispositif selon la revendication 1, où le premier et le deuxième bras (110 ; 210) sont contraints vers la configuration de réception de tissu.

10. Dispositif selon l'une des revendications précédentes,
où la première et la deuxième découpes angulaires (112, 114) sont formées sur des parois latérales opposées d'un élément de fixation (106) de la pince (102) et définissent des ouvertures en rainure sur l'élément de fixation (106).

11. Dispositif d'hémostase, comprenant :
un dispositif de préhension (100 ; 200) selon la revendication 1 ; et
un élément d'hémostase (241) prévu sur le dispositif de préhension, ledit élément d'hémostase (241) étant formé pour sceller un tissu cible et maintenir un joint sur celui-ci après le retrait du dispositif (100 ; 200) hors du corps.

12. Dispositif selon la revendication 11, où l'élément d'hémostase (241) est une bande élastique (241) étirée sur le manchon extérieur (140 ; 240), ladite bande élastique (241) pouvant coulisser axialement le long du dispositif de préhension et étant déployée depuis une extrémité distale du dispositif de préhension sur le tissu saisi par le dispositif de préhension, de sorte que ladite bande élastique (241) est resserrée autour du tissu saisi.

13. Dispositif selon la revendication 11,
où ledit dispositif comprend en outre un élément support (230) monté à l'intérieur de la pince (102) et comportant une tige allongée (232) s'étendant vers une extrémité évasée (234) à son extrémité distale, l'extrémité évasée (234) pouvant s'étendre distalement au-delà des extrémités distales du premier et du deuxième bras (110 ; 210) pour faciliter la traction du tissu entre le premier et le deuxième bras (110 ; 210), ou
où les extrémités du premier et du deuxième bras (110 ; 210) présentent des ardillons (128) ou des pointes ou des pointes émoussées.

14. Dispositif selon la revendication 11, où les parois extérieures du premier et du deuxième bras (110 ; 210) présentent une pluralité de crans (132) formés pour permettre au manchon extérieur (140 ; 240) de coulisser distalement sur ceux-ci et empêcher le manchon extérieur (140 ; 240) de se déplacer proximalement au-delà des crans (132).

15. Dispositif selon la revendication 14,
où une paroi intérieure du manchon extérieur (140 ; 240) présente une pluralité de dentures (138) positionnées pour retenir les crans (132) par engrènement.

16. Dispositif selon la revendication 11, où le premier et le deuxième bras (110 ; 210) sont contraints par ressort vers la configuration de réception de tissu.
